# EUROPEAN PATENT SPECIFICATION

(11) **EP 3 187 500 B1**
(45) Date of publication and mention of the grant of the patent: **02.01.2019**
(21) Application number: 15836991.8
(22) Date of filing: 25.08.2015
(51) Int. Cl.: C07D 493/04, C07F 7/18, C07D 493/22, C07B 61/00

(54) **METHOD FOR PRODUCING ANTITUMOR AGENT USING A HOMOGENIZER**
VERFAHREN ZUR HERSTELLUNG EINES ANTITUMORMITTELS MIT EINEM HOMOGENISATOR
PROCÉDÉ DE PRODUCTION D'UN AGENT ANTITUMORAL À L'AIDE D'UN HOMOGÉNÉISATEUR

(30) Priority: 27.08.2014 JP 2014173033
(43) Date of publication of application: 05.07.2017
(73) Proprietor: Eisai R&D Management Co., Ltd., Tokyo 112-8088 (JP)
(72) Inventor: CHIBA, Hiroyuki, Kamisu-shi Ibaraki 314-0255 (JP); FUKUYAMA, Takashi, Tsukuba-shi Ibaraki 300-2635 (JP); TAKIGAWA, Teiji, Tsukuba-shi Ibaraki 300-2635 (JP)
(74) Representative: Hoffmann Eitle
(86) International application number: PCT/JP2015/073787
(87) International publication number: WO 2016/031796

(56) References cited:
- JP-A- H10 128 113
- JP-A- 2003 513 068
- JP-A- 2010 285 428
- JP-A- 2011 504 166
- FUKUYAMA T. ET AL.: "Application of a Rotor-Stator High Shear System for Cr/Mn-Mediated Reactions in Eribulin Mesylate Synthesis", ORGANIC PROCESS RESEARCH AND DEVELOPMENT, vol. 20, 21 December 2015 (2015-12-21), pages 100-104, XP002776007,

## Description

### Technical Field

This invention relates to methods for industrial manufacturing of an anticancer agent

### Background Art

Eribulin represented by a following formula (I) is a microtubule dynamics inhibitor which inhibits microtubule elongation and arrests cell division, and is used as a medicine for treatment of breast cancer.

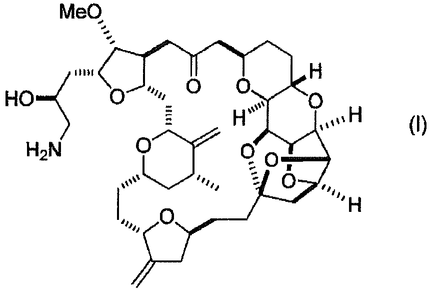

Eribulin and pharmaceutically acceptable salt thereof and methods for manufacturing them are disclosed in Patent Literature 1. And methods for manufacturing Eribulin and its methanesulfonic acid salt are disclosed in Patent Literature 2 and 3. In Patent Literature 4, novel intermediates for manufacturing of Eribulin and its methanesulfonic acid salt, and in Patent Literature 4 and 5, methods using novel desulfonylation reaction for application to these intermediates are disclosed.

### Citation List

### Patent Literature

Patent Literature 1: International Publication WO 99/065894
Patent Literature 2: International Publication WO 2005/118565
Patent Literature 3: International Publication WO 2011/094339
Patent Literature 4: International Publication WO 2009/064029
Patent Literature 5: Japanese Patent Application Laid-Open Publication No. 2010-285428

### Summary of Invention

### Technical Problem

One of objectives of this invention is to provide methods for manufacturing Eribulin and Eribulin mesylate effectively and in good yield.

### Solution to Problem

As results of extensive research efforts to achieve the foregoing objectives, the present inventors have found that the reaction has been completed promptly with stirring the reaction system by a homogenizer, and accomplished this invention.

Specifically, the present invention relates to:
[1] A method for manufacturing a compound represented by a formula (II) or salt thereof 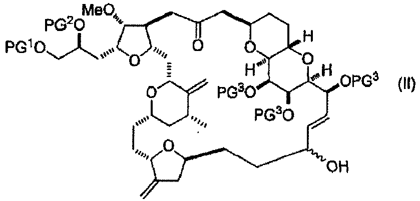
   wherein PG¹, PG² and PG³ each independently represent a protective group of a hydroxyl group
   from a compound represented by a formula (III), 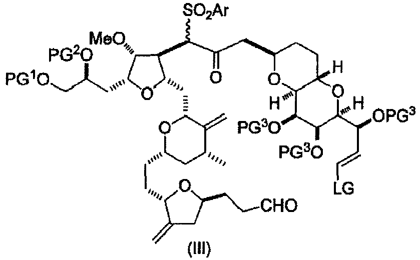
   wherein Ar represents an aryl group which may have substituent groups or a heteroaryl group which may have substituent groups, PG¹, PG² and PG³ each independently represent a protective group of a hydroxyl group and LG represents a leaving group,
   comprising a reaction step where a reactant is stirred with homogenizer, in the presence of manganese powder and chromium compound,
[2] The method according to [1], wherein the chromium compound is chromium chloride (III),
[3] The method according to [1] or [2], wherein the step where the reactant is stirred with homogenizer in the presence of manganese powder and chromium compound is a step for manufacturing a compound represented by a formula (IV), 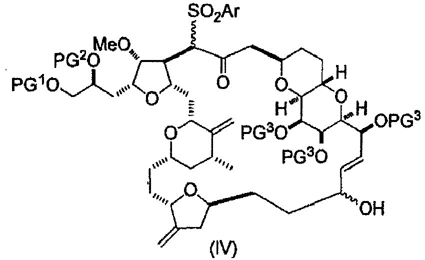
   wherein Ar represents an aryl group which may have substituent groups or a heteroaryl group which may have substituent groups, PG¹, PG² and PG³ each independently represent a protective group of hydroxyl group,
   from a compound represented by a formula (III), 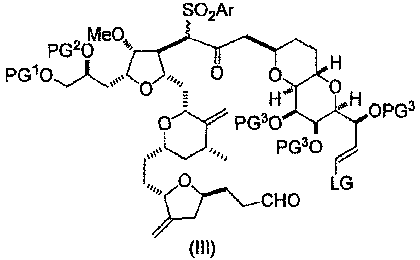
   wherein Ar represents an aryl group which may have substituent groups or a heteroaryl group which may have substituent groups, PG¹, PG² and PG³ each independently represent a protective group of a hydroxyl group and LG represents a leaving group,
[4] The method according to any one of [1] to [3], wherein the step where the reactant is stirred with homogenizer in the presence of manganese powder and chromium compound is a step for manufacturing a compound represented by a formula (II), 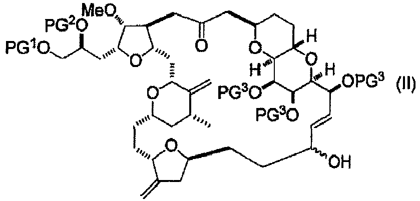
   wherein PG¹, PG² and PG³ each independently represent a protective group of hydroxyl group,
   from a compound represented by a formula (IV), 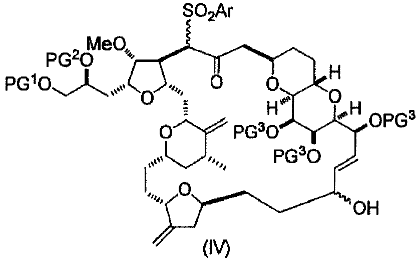
   wherein Ar represents an aryl group which may have substituent groups or a heteroaryl group which may have substituent groups, PG¹, PG² and PG³ each independently represent a protective group of hydroxyl group,
[5] The method according to [1] or [2], wherein the step where the reactant is stirred with homogenizer in the presence of manganese powder and chromium compound is a step for manufacturing a compound represented by a formula (II), 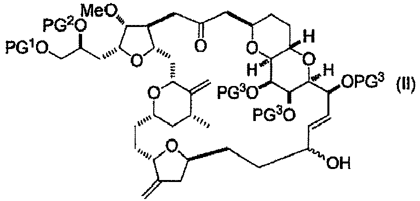
   wherein PG¹, PG² and PG³ each independently represent a protective group of hydroxyl group,
   from a compound represented by a formula (V), 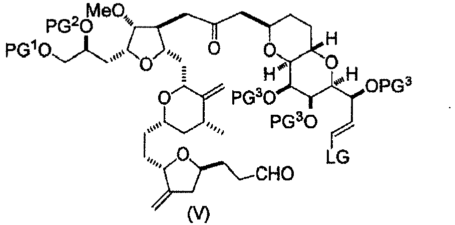
   wherein PG¹, PG² and PG³ each independently represent a protective group of a hydroxyl group and LG represents a leaving group,
[6] The method according to [3] or [5], wherein conducting the reaction in the presence of nickel compound in the reaction step,
[7] The method according to any one of [1] to [6], wherein catalytic amount of chromium compound is used,
[8] The method according to any one of [1] to [7], wherein PG¹, PG² and PG³ are tert-butyldimethylsilyl (TBS) group,
[9] A method for manufacturing Eribulin or the salt thereof comprising the method according to the any one of [1] to [8],
[10] The method according to [9], wherein the salt of Eribulin is Eribulin mesylate.

### Advantageous Effects of Invention

Methods of the present invention for manufacturing of Eribirlin and salt thereof achieve completion of the reaction promptly even though the reaction scale is far more than the laboratory scale, and can manufacture the Eribulin and salt thereof efficiently and in good yield.

### Description of Embodiments

Now, the definitions of symbols and terms used herein are described in details.

A "C₆₋₁₀ aryl group" used in this specification means an aromatic hydrocarbon group having 6 to 10 carbon atoms. Exemplary such C₆₋₁₀ aryl groups include a phenyl group, a 1-naphthyl group and a 2-naphthyl group, and it is preferably a phenyl group.

A "C₁₋₉ heteroaryl group" used in this specification means an aryl group having 1 to 9 carbon atoms forming a ring and having 1 to 5 hetero atoms as atoms forming the ring, exemplary such groups include a furyl group, a thienyl group, a pyrrolyl group, an imidazolyl group, a triazolyl group, a tetrazolyl group, a thiazolyl group, a pyrazolyl group, an oxazolyl group, an isoxazolyl group, an isothiazolyl group, a furazanyl group, a thiadiazolyl group, an oxadiazolyl group, a pyridyl group, a pyrazinyl group, a pyridazinyl group, a pyrimidinyl group a triazinyl group, a purinyl group, a pteridinyl group, a quinolyl group, an isoquinolyl group, a naphthidinyl group, a quinoxalinyl group, a cinnolinyl group, a quinazolinyl group, aphthalazinyl group, an imizazopyridyl group, an imizazothiazolyl group, an imidazoxazolyl group, a benzothiazolyl group, a benzoxazolyl group, a benzimidazolyl group, an indolyl group, an isoindolyl group, an indazolyl group, a pyrrolopyridyl group, a thienopyridyl group, a furopyridyl group, a benzothiadiazolyl group, a benzoxadiazolyl group, a pyridopyrimidinyl group, a benzofuryl group, a benzothienyl group, a benzo[1,3]dioxolyl group, a thienofuryl group, a chromenyl group, a chromanyl group, a cumarinyl group, a quinolonyl group and the like, and it is preferably a pyridyl group, a pyrazinyl group, a quinolyl group, a quinoxalinyl group, a benzothiazolyl group, a cumarinyl group, a quinolonyl group and the like, more preferably a pyridyl group, a quinolinyl group, a quinoxalinyl group and the like.

The description, "may have substituent groups" in this specification means that it is not substituted with any substituent groups or substituted with some substituent groups. The substituent groups are not particularly limited, but exemplary such substituent groups include a halogen atom such as a fluorine atom, a chlorine atom and the like, a C₁₋₆ alkyl group such as a methyl group, an ethyl group, a n-propyl group, an isopropyl group and the like, a C₃₋₆ cycloalkyl group such as a cyclopropyl group, a cyclohexyl group and the like, and a C₁₋₆ alkoxy group such as a methoxy group, an ethoxy group, a n-propoxy group, an isopropoxy group and the like, and it is preferably unsubstituted.

Exemplary such "a protective group of a hydroxyl group" used in this specification includes protective groups described in "Protecting Groups in Organic Synthesis, Greene 4th edition, John Wiley & Sons, Inc.". In specific embodiments, PG¹, PG² and PG³ are independently selected, as a group containing the oxygen atom to which they are attached, from esters, ethers, silylethers, alkylethers, aralkylethers, and alkoxyalkylethers. Examples of the esters include formates, acetates, carbonates, and sulfonates. Specific examples thereof include formate, benzoylformate, acetate, chloroacetate, trifluoroacetate, methoxyacetate, triphenylmethoxyacetate, p-chlorophenoxyacetate, 3-phenylpropionate, 4-oxopentanoate, 4,4-(ethylenedithio)pentanoate, pivaloate (Piv), vinylpivaloate, crotonate, 4-methoxy-crotonate,1-naphthoate, 2-naphtoate, benzoate, p-methoxybenzoate, 2,4,6-trimethylbenzoate, methylcarbonate, 9-fluorenylmethycarbonatel, ethylcarbonate, 2,2,2-trichloroethylcarbonate, 2-(trimethylsilyl)ethylcarbonate, 2-(phenylsulfonyl)ethylcarbonate, vinylcarbonate, allylcarbonate, tert-butylcarbonate p-nitrobenzylcarbonate, methyl ether, tert-butyl ether, allyl ether , methoxymethyl ether, methylthiomethyl ether, (2-methoxyethoxy)methyl ether, β-(trimethylsilyl)ethoxymethyl ether, benzyl ether, p-methoxybenzyl ether (MPM), 3,4-dimethoxybenzyl ether, triphenylmethyl ether (trityl), o-nitrobenzyl ether, p-nitrobenzyl ether, p-halobenzyl ether, 2,6-dichlorobenzyl ether, p-cyanobenzyl ether, naphtylmethyl ether, 2-picolyl ether, 4-picolyl ether, trimethylsilyl ether (TMS), triethylsilyl ether (TES), tert-butyldimethylsilyl ether (TBS), tert-butyldiphenylsilyl ether (TBDPS), triisopropylsilyl ether (TIPS), triphenylsilyl ether (TPS), benzyloxymethyl ether and the like. It is preferably tert-butyldimethylsilyl ether (TBS). And, PG1 and PG2 may form a diol protective group such as acetal or ketal together with the oxygen atom to which they are attached. Examples of the diol protective group include methylene, ethylidene, benzylindene, isopropylidene, cyclohexylidene, and cyclopentylindene.

A "leaving group" used in this specification means a sulfonate group, a halogen atom or the like. Exemplary such leaving groups include a mesylate, a tosylate, a triflate, a chloromethanesulfonate, a chlorine atom, a bromine atom, an iodide atom and the like, and it is preferably an iodide atom.

The compound (I) or salt thereof may be an anhydride, a hydrate or a solvate.

The salt of the compound (I) is not limited. Exemplary such salts include a salt with inorganic acid, salts with organic acid, salt with acidic amino acid, or the like.

Specific preferable examples of such salts with inorganic acid include a salt with a hydrochloric acid, a hydrobromic acid, a sulfuric acid, a nitric acid or a phosphoric acid.

Specific preferable examples of such salts with organic acid include a salt with an acetic acid, a succinic acid, a fumaric acid, a maleic acid, a tartaric acid, a citric acid, a lactic acid, a stearic acid, a benzoic acid, a methanesulfonic acid, an ethanesulfonic acid, p-toluenesulfonic acid and the like, and it is preferably a salt with methanesulfonic acid.

Specific preferable examples of such salts with acidic amino acid include a salt with an aspartic acid and a glutamic acid.

One embodiment of this invention is shown as a following scheme. 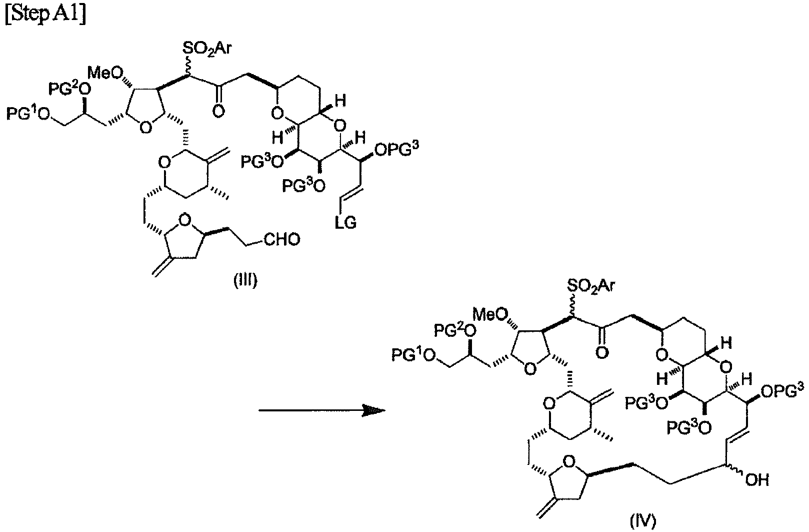 wherein the scheme, Ar represents a C₆₋₁₀ aryl group which may have substituent groups or a C₁₋₉ heteroaryl group which may have substituent groups, PG¹, PG² and PG³, each independently, represent a protection group of hydroxyl group and LG represents a leaving group.

Another embodiment of this invention is shown as a following scheme, 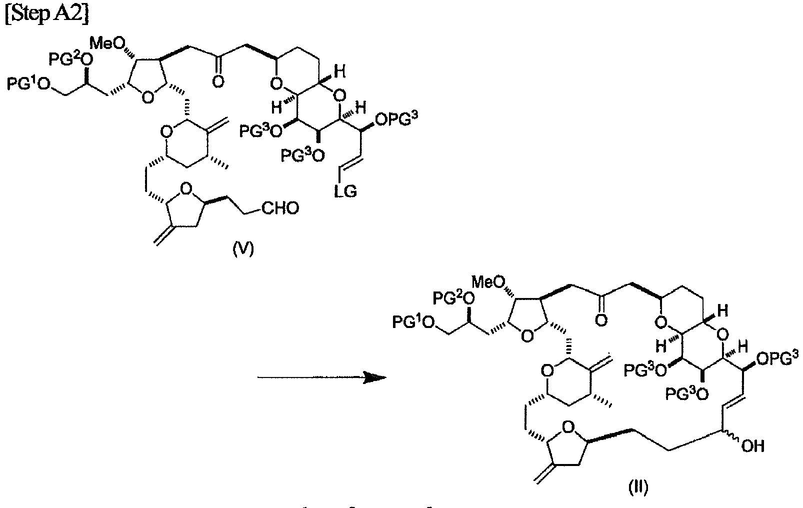 wherein the scheme, PG¹, PG² and PG³, each independently, represent a protection group of hydroxyl group and LG represents a leaving group.

The step A1 or A2 is a step to manufacture the compound represented by the formula (IV) or (II) conducting a intramolecular cyclization reaction of the compound represented by the formula (III) or (V) stirring with homogenizer in the presence of manganese powder, chromium compound and nickel compound

A solvent used in this step is not particularly limited as long as it dissolves a starting material to some extent and does not inhibit the reaction, and exemplary such solvents include a toluene, a tetrahydrofuran a dimethoxyethane, a methyl tert-butyl ether, a dimethylformamide, a methanol, and an acetonitrile. The solvent can be used alone or as the mixture of the two or more solvents. A mixture of water and one or more solvents also can be used as a solvent of this invention. The amount of the solvent is not particularly limited, and arbitrary amount of the solvent can be used as long as the reaction proceeds.

A chromium compound is used in the reaction of this step. A trivalent chromium compound or a divalent chromium compound can be used, and both of an organic chromium compound or an inorganic chromium compound also can be used. It is preferably an inorganic chromium compound More preferable chromium compound is a halogenated chromium, a chromium carboxylate (III) or a chromium carboxylate (II), which is represented by the formula Cr(III)X₃ or Cr(II)X₂, wherein X represents a halogen atom or organo-carboxylate. X is preferably chlorine atom or bromine atom. Particularly preferable trivalent chromium compound is CrCl₃ anhydride, CrCl₃/H₂O, CrBr₃/6H₂O, CrCl₃/3THF or Cr(OAc)₃, wherein Ac represents an acetyl group. And particularly preferable divalent chromium compound is CrCl₂ or Cr(OAc)₂, wherein Ac represents an acetyl group. The amount of the trivalent or divalent chromium compound can be determined arbitrarily so as to permit the reaction to proceed, and it is not particularly limited. 0.01 to 10 equivalent amount for compound represented by the formula (III) or (V) can be used. Catalytic amount is preferable, 0.05 to 0.5 equivalent is more preferable, and 0.05 to 0.2 equivalent is particularly preferable.

Manganese powder is used with the aforesaid chromium compound in the reaction of this step. The amount of the manganese powder can be determined arbitrarily so as to permit the reaction to proceed, and it is not particularly limited. 1 to 10 equivalent amount for compound represented by the formula (III) or (V) can be used. 2 to 5 equivalent is preferable, and 3 to 5 equivalent is particularly preferable.

The reaction system is stirred with homogenizer in the reaction of this step. A "homogenizer" used in this specification means an equipment which can add mechanical shearing action to liquid-solid or liquid-liquid phasic flow by a rotary blade, ultra sonication, pressure, high-frequency wave or the like. But it is not particularly limited as long as it can produce a well-established dispersion effect and breakup effect, such as a dispersion pump and a breakup pump is also included. Preferably, it is a homogenizer which uses the rotary blade. Various homogenizers which can control circumferential velocity of the rotary blade within a range of about 0 to 40 m/s are available. Circumferential velocity of the rotary blade of the homogenizer to stir can be determined arbitrarily so as to permit the reaction to proceed, and it is not particularly limited, it is preferably more than 1.0 m/s, particularly preferably more than 2.0 m/s. Considering dispersion effect and breakup effect, high circumferential velocity is preferable, but the maximum circumferential velocity is about 10 to 40 m/s in a usually obtainable homogenizer.

The reaction is conducted with a nickel compound in this step. As a nickel compound, NiCl₂ or a nickel chloride complex, such as NiCl2/DME, NiCl₂/2,9-dimethyl-1,10-phenanthroline complex (Ni-neo complex) or the like are exemplified. Ni-neo complex can be prepared with N₁Cl₂ or N₁Cl_{2/}DME and 2,9-dimethyl-1,10-phenanthroline in advance and can be used in the reaction. The amount of the nickel compound can be determined arbitrarily so as to permit the reaction to proceed, and it is not particularly limited, it is preferably 0.01 to 0.5 equivalent amount for compound represented by the formula (III) or (V), more preferably 0.05 to 0.2 equivalent.

Use of a ligand is preferable in this reaction. The ligand used in this step is not particularly limited so as to permit the reaction to proceed, it include such 4-4'-dialkyl/diaryl-2,2'-bipyridyl, 3,3'-dialkyl-2,2'-bipyridyl, a kind of phenanthroline or hydroxyqunoline, and the like. It is exemplified such as 4,4'-di-tert-butyl-2,2'-bipyridyl, 4,4'-diphenyl-2,2'-bipyridyl, 4,4'-dinonyl-2,2'-bipyridyl, 3,3'-dimethyl-2,2'-bipyridyl, 8-hydroxyquinoline, 4,7-diphenyl-1,10-phenanthroline or the like, it is particularly preferably 4,4'-di-tert-butyl-2,2'-bipyridyl. The amount of the ligand can be determined arbitrarily so as to permit the reaction to proceed, and it is not particularly limited, it can be used 0.01 to 10 equivalent amount for compound represented by the formula (III) or (V), it is preferably 0.05 to 0.5 equivalent, more particularly preferably 0.05 to 0.2 equivalent

A metallocene compound can further be added to this reaction. The reaction can progress even though the amount of the chromium compound for the compound represented by the formula (III) or (V) is less than one equivalent by using the metallocene compound. Exemplary such the metallocene compounds include zirconium compounds including a bis(mono or poly alkyl substituted cyclopentadienyl)zirconium dichloride such as a bis(cyclopentadienyl)zirconium dichloride, a bis(methylcyclopentadienyl)zirconium chloride and a bis(pentamethylcyclopentadienyl)zirconium chloride, a bis(indenyl)zirconium dichloride, a bis(mono or poly alkyl substituted indenyl)zirconium dichloride and the like, and titanium or hafnium compounds which have a chemical structure in which a zirconium atom of metallocene compounds is replaced by a titanium or a hafnium atom. As the metallocene compound used for this invention, a zirconium compound is preferable and a bis(cyclopentadienyl)zirconium dichloride is particularly preferable. Instead of the metallocene compound, a halogenated silyl compound such as trimethylsilyl chloride also can be used in the reaction. The amount of these compounds can be determined arbitrarily so as to permit the reaction to proceed, and it is not particularly limited. It can be used 1 to 5 equivalent amount for compound represented by the formula (III) or (V), it is preferably 1 to 2 equivalent, particularly preferably 1 to 1.5 equivalent

The reaction can be carried out at 5 to 50 °C, preferably 15 to 40 °C, more preferably 25 to 35 °C, but the reaction temperature is not particularly limited. Furthermore, it is preferable to conduct the reaction under inactive gas such as nitrogen or argon.

Another embodiment of this invention is shown as a following scheme, 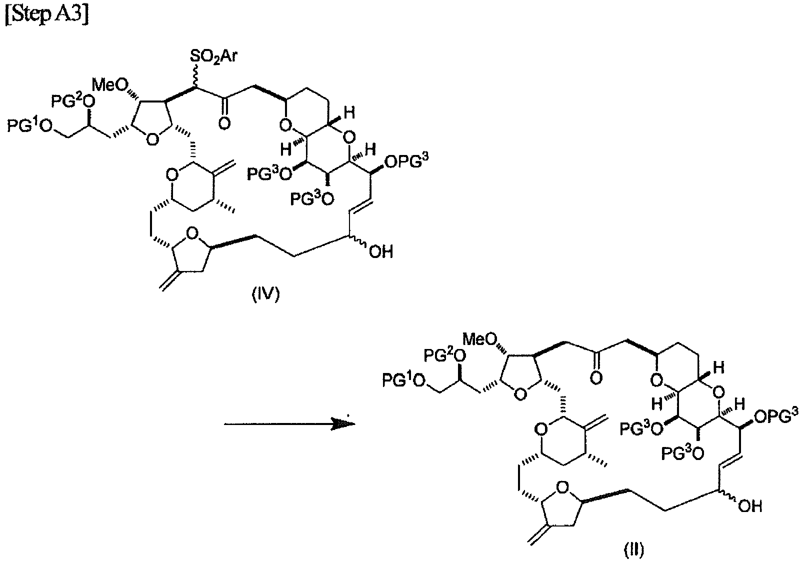 wherein the scheme, Ar represents a aryl group which may have substituent groups or a heteroaryl group which may have substituent groups, PG¹, PG² and PG³, each independently, represent a protection group of hydroxyl group.

This is a step to manufacture the compound represented by the formula (II) conducting a desulfonylation reaction of the compound represented by the formula (IV) stirring with homogenizer in the presence of manganese powder and chromium compound.

A solvent used in this step is not particularly limited as long as it dissolves a starting material to some extent and does not inhibit the reaction, and exemplary preferred solvents include a toluene, a tetrahydrofuran a dimethoxyethane, a methyl tert-butyl ether, a dimethylformamide, a methanol, and an acetonitrile. The solvent can be used alone or as the mixture of the two or more solvents. A mixture of water and one or more solvents also can be used as a solvent of this invention. Particularly preferably, the solvent is a tetrahydrofuran, a methanol, an acetonitrile, a dimethylformamide and a mixture of two or more solvents as above. The amount of the solvent is not particularly limited, and arbitrary amount of the solvent can be used as long as the reaction proceeds.

A chromium compound is used in the reaction of this step. A trivalent chromium compound or a divalent chromium compound can be used, and both of an organic chromium compound or an inorganic chromium compound also can be used. It is preferably an inorganic chromium compound. More preferable chromium compound is a halogenated chromium, a chromium carboxylate (III) or a chromium carboxylate (II), which is represented by the formula Cr(III)X₃ or Cr(II)X₂, wherein X represents a halogen atom or organo-carboxylate. Particularly preferable trivalent chromium compound is CrCl₃ anhydride, CrCl₃/6H₂O, CrBr₃/6H₂O, CrCl₃/3THF, or Cr(OAc)₃, wherein Ac represents an acetyl group. And particularly preferable divalent chromium compound is CrCl₂ or Cr(OAc)₂, wherein Ac represents an acetyl group. The amount of the trivalent or divalent chromium compound can be determined arbitrarily so as to permit the reaction to proceed, and it is not particularly limited. 0.1 to 5 equivalent amount for compound represented by the formula (IV) can be used, 0.5 to 3 equivalent is preferable and 1 to 2 equivalent is particularly preferable.

Manganese powder is used with the aforesaid chromium compound in the reaction of this step. The amount of the manganese powder can be determined arbitrarily so as to permit the reaction to proceed, and it is not particularly limited. 1 to 20 equivalent amount for compound represented by the formula (IV) can be used, 1 to 10 equivalent is preferable, and 2 to 5 equivalent is particularly preferable.

The reaction system is stirred with homogenizer in this reaction step. A "homogenizer" used in this specification means an equipment which can add mechanical shearing action to liquid-solid or liquid-liquid phasic flow by a rotary blade, ultra sonication, pressure, high-frequency wave or the like. And it is not particularly limited as long as it can produce a well-established dispersion effect and breakup effect, such as a dispersion pump and a breakup pump is also included. It is preferably a homogenizer which uses the rotary blade. Various homogenizers which can control circumferential velocity of the rotary blade within a range of about 0 to 40 m/s are available. Circumferential velocity of the rotary blade of the homogenizer to stir can be determined arbitrarily so as to permit the reaction to proceed, and it is not particularly limited, it is preferably more than 1.0 m/s, particularly preferably more than 2.0 m/s. Considering dispersion effect and breakup effect, high circumferential velocity is preferable, but the maximum circumferential velocity is about 10 to 40 m/s in a usually obtainable homogenizer.

Use of a ligand is preferable in this reaction. The ligand used in this step is not particularly limited so as to permit the reaction to proceed, and it include 4-4'-dialkyl/diaryl-2,2'-bipyzidyl, 3,3'-dialkyl-2,2'-bipyridyl, a kind of phenanthroline or hydroxyqunoline and the like. It is exemplified such as 4,4'-di-tert-butyl-2,2'-bipyridyl, 4,4'-diphenyl-2,2'-bipyridyl, 4,4'-dinonyl-2,2'-bipyridyl, 3,3'-dimethyl-2,2'-bipyridyl, 8-hydroxyquinoline, 4,7-diphenyl-1,10-phenanthroline or the like, it is particularly preferably 4,4'-di-tert-butyl-2,2'-bipyridyl. The amount of the ligand can be determined arbitrarily so as to permit the reaction to proceed, and it is not particularly limited, it can be used 0.1 to 5 equivalent amount for compound represented by the formula (IV), it is preferably 0.5 to 3 equivalent, particularly preferably 1 to 2 equivalent

A metallocene compound can further be added to this reaction. The reaction can progress even though the amount of the chromium compound for the compound represented by the formula (IV) is less than one equivalent by using the metallocene compound. Exemplary such the metallocene compounds include zirconium compounds including a bis(mono or polyalkyl substituted cyclopentadienyl)zirconium dichloride such as a bis(cyclopentadienyl)zirconium dichloride, a bis(methylcyclopentadienyl)zirconium chloride and a bis(pentamethylcyclopentadienyl)zirconium chloride, a bis(indenyl)zirconium dichloride, a bis(mono or polyalkyl substituted indenyl)zirconium dichloride and the like, and titanium or hafnium compounds which have a chemical structure in which a zirconium atom of metallocene compounds is replaced by a titanium or a hafnium atom. As the metallocene compound used for this invention, a zirconium compound is preferable and a bis(cyclopentadienyl)zirconium dichloride is particularly preferable. Instead of the metallocene compound, a halogenated silyl compound such as trimethylsilyl chloride also can be used in the reaction. The amount of these compounds can be determined arbitrarily so as to permit the reaction to proceed, and it is not particularly limited, it can be used 1 to 5 equivalent amount for compound represented by the formula (IV), it is preferably 1 to 2 equivalent.

The reaction can be done at the reaction temperature 5 to 50 °C, preferably 15 to 40 °C, more preferably 25 to 35 °C, but it is not particularly limited. Furthermore, it is preferable to conduct the reaction under inactive gas such as nitrogen or argon.

Another embodiment of this invention is a method for manufacturing the compound represented by the formula (VII), after the aforementioned step A1 and/or A3 or A2, further conducting an oxidation step shown as the following step B for the compound represented by the formula (II). 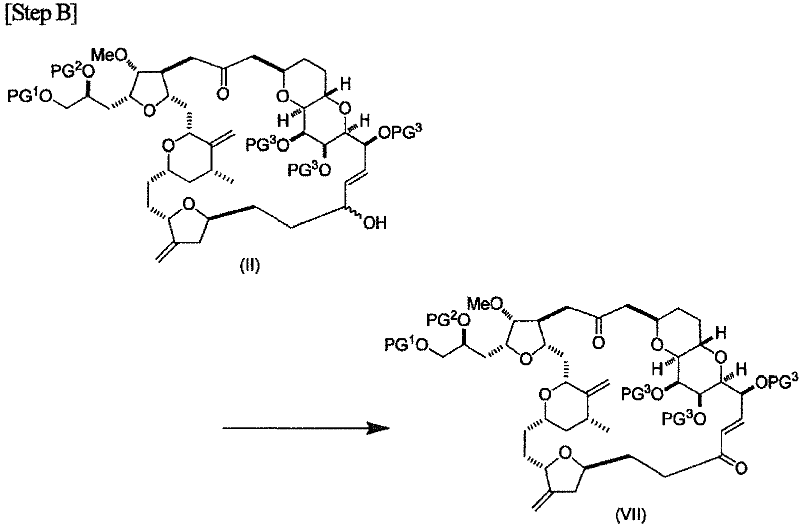 wherein the scheme, PG¹, PG² and PG³, each independently, represent a protection group of hydroxyl group.

Another embodiment of this invention is a method for manufacturing the compound represented by the formula (VIII), after the aforementioned step A1 and/or A3 or A2, and B, further conducting a deprotection reaction of the protecting group of hydroxyl group as the following step C for the compound represented by the formula (VII): The protection group can be removed arbitrarily by the known method disclosed in Green's PROTECTIVE GROUP IN ORGANIC CHEMISTRY fourth edition, JOHN WILEY & SONS, INC or the like. 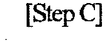 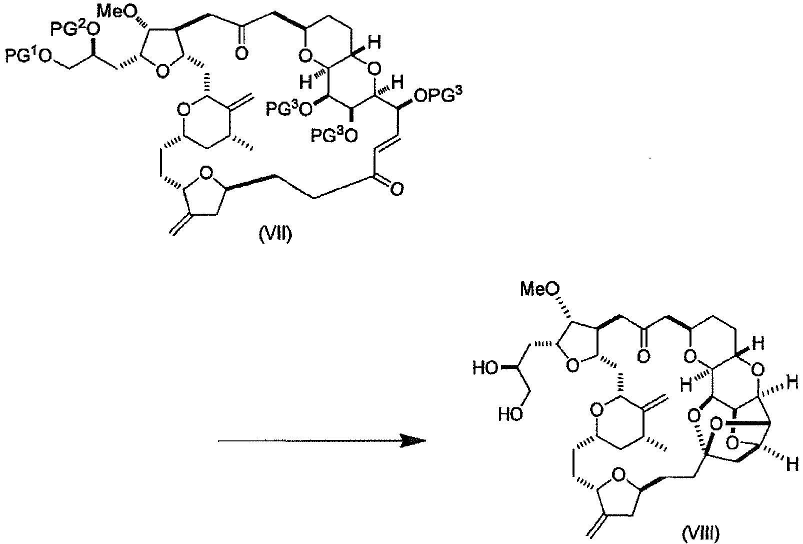 wherein the scheme, PG¹, PG² and PG³, each independently, represent a protection group of hydroxyl group.

Another embodiment of this invention is a method for manufacturing the compound represented by the formula (IX), after the aforementioned step A1 and/or A3 or A2, B and C, further conducting an amination reaction of step D for the compound represented by the formula (VIII).

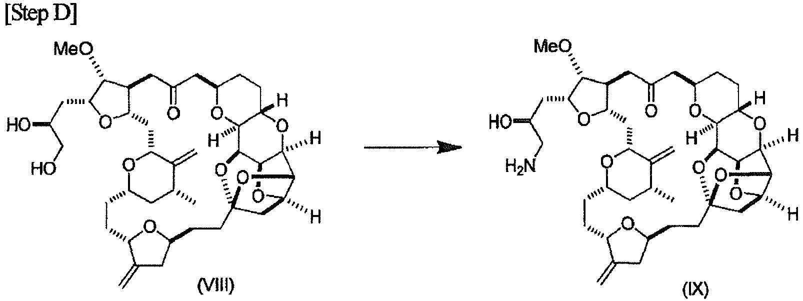

Another embodiment of this invention is a method for manufacturing Eribulin mesylate represented by the formula (X), after the aforementioned step A1 and/or A3 or A2, then B, C and D, further conducting a salification of the compound represented by the formula (IX) as shown following Step E.

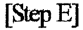

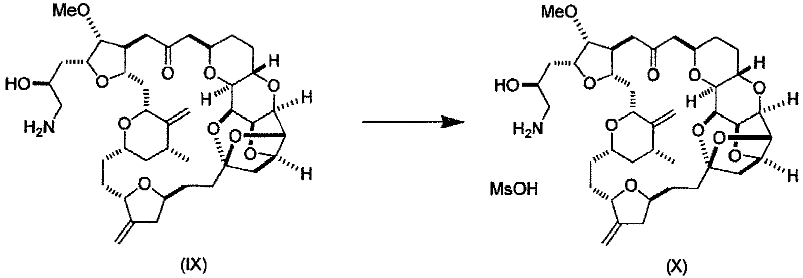

In this step, the compound represented by the formula (IX) is treated with MsOH and NH₄OH in water and acetonitrile, then, concentrated residue of the reaction mixture is dissolved with DCM and pentane, then adding absolute pentane to the solution and precipitated residue is filtered and dried under high reduced pressure, can afford Eribulin mesylate.

Aforementioned manufacturing methods of step B, C, D and E also can be conducted according to methods described in WO99/065894, WO2005/118565 and WO2011/094339.

Hereinafter, the manufacturing methods of this invention are described circumstantially.

The compound represented by the following formula (III) which is one of starting material of the manufacturing methods can be obtained according to methods described in WO99/065894, WO2005/118565, WO2009/064029 and WO2011/094339. 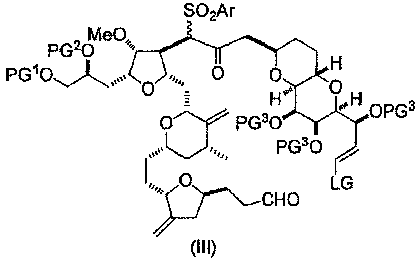 wherein the scheme, Ar represents an aryl group which may have substituent groups or a heteroaryl group which may have substituent groups, PG¹, PG² and PG³, each independently, represent a protection group of hydroxyl group, LG represents a leaving group.

And the compound represented by the following formula (IV) which is one of reaction substrates of the manufacturing methods also can be obtained according to methods described in WO99/065894, WO2005/118565, and WO2009/064029. 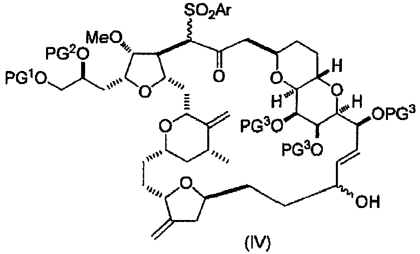 wherein the scheme, Ar represents an aryl group which may have substituent groups, or a heteroaryl group which may have substituent groups, PG¹, PG² and PG³, each independently, represent a protection group of hydroxyl group.

Furthermore, the compound represented by the following formula (V) which is one of reaction substrates of the manufacturing methods can be obtained according to methods described in WO99/065894 and WO2005/118565.

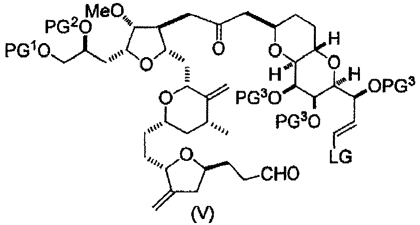

In case both PG¹, PG² and PG³ are tert-butyldimethylsilyl ether (TBS) in the formula (II), it corresponds to the compound represented by the following formula (VI).

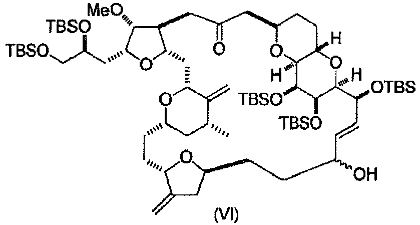

### Example

Hereinafter, it is explained circumstantially with examples, but this invention is not limited to the examples. In this specification, the following abbreviations are used.
ACN: Acetonitrile
DCM: Dichloromethane
DME: Dimethoxyethane
IPA: Isopropanol
MTBE: Methyl tert-butyl ether
TBAF: Tetrabutylammonium fluoride
THF: Tetrahydrofuran
Ts2O: p-Toluenesulfonic acid anhydride
PPTS: Pyridinium p-toluenesulfonate

### [Reference example 1] Manufacturing of Ni-neo complex

To a solution of 2,9-dimethyl-1,10-phenanthroline 0.5 hydrate (23.7g) in acetonitrile (1475mL), was added NiCl₂/DME(25.0g), and the mixture was stirred for 1.5 hours. A precipitated solid was collected by filtration and dried to afford the Ni-neo complex (30.8g).

### [Example 1] Manufacturing of ER-413207

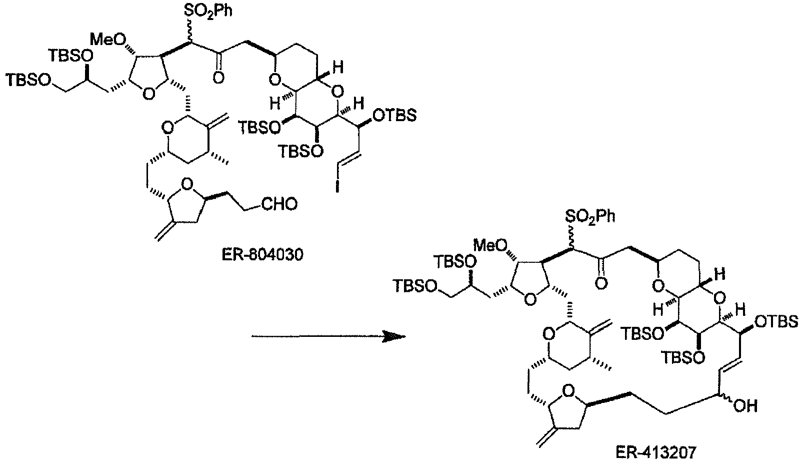

A homogenizer (Body; IKA T25 digital ULTRA TURRAX, Shaft; IKA S25N-25F) was used to stir the reaction.

In a glove box, to a reaction vessel with chromium chloride (III) (24.9 mg), 4,4'-di-tert-butyl-2,2'-bipyridyl (42.2 mg), zirconocene chloride (506.1 mg) and manganese (powder, particle size; 75 micrometer, 345.9 mg), tetrahydrofuran (25 mL) was added, and then stirred for 30 minutes by the homogenizer with circumferential velocity 2.85 m/s. To the reaction mixture, Ni-neo complex (53.2 mg) was added and stirred for 15 minutes by the homogenizer with circumferential velocity 2.85 m/s. To the reaction mixture, a solution of ER-804030 (2.5 g, 1.59 mmol) in tetrahydrofuran (75 mL) was added dropwise over 15minutes at room temperature. After completion of addition, the mixture was stirred for 1 hour with circumferential velocity 5.34 m/s at the same temperature. To the reaction mixture, heptane (50 mL) was added, and then the reaction vessel was gotten out of the glove box. To the reaction mixture, 10% citric acid aqueous solution (25 mL) was added and sirred for 40 minutes at room temperature. The aqueous layer was removed and the layer was re-extracted with heptane. The organic layers were combined and washed with aqueous solution of 5% sodium bicarbonate and 10% sodium sulfate (25mL). The organic layer was concentrated to afford ER-413207 (2.0 g, 1.38 mmol, yield 86.8%) and ER-118047/48 (162.2 mg, 0.124 mmol, yield 7.8%).

### [Example 2] Manufacturing of ER-413207

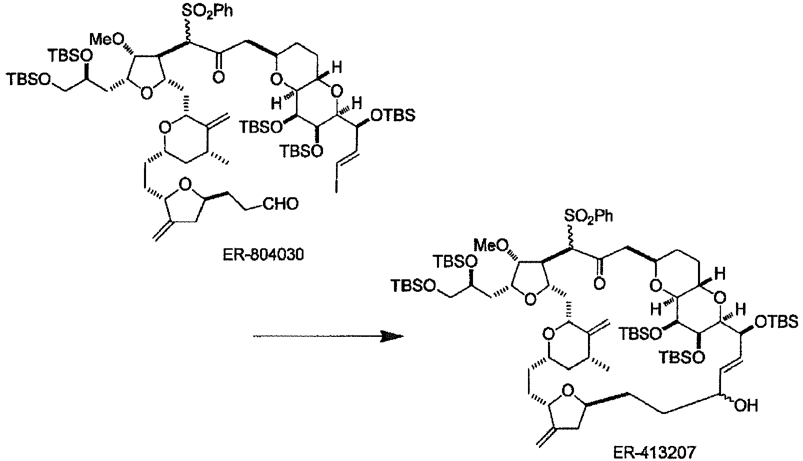

A homogenizer (Body; IKA T65 digital ULTRA TURRAX, Shaft; IKA S65KG-HH-G65F) was used to stir the reaction.

A reaction vessel with chromium chloride (III) (3.66 g), 4,4'-di-tert-butyl-2,2'-bipyridyl (620 g), zirconocene dichloride (74.3 g) and manganese (powder, particle size;75 micrometer, 50.8 g) was filled with argon gas. To the reaction vessel, tetrahydrofuran (7340 mL) was added, and then stirred for 1 hour by the homogenizer with circumferential velocity 2.67 m/s. To the reaction mixture, a suspension of Ni-neo complex (7.80 g) in tetrahydrofuran (367 mL) was added and stirred for 5 minutes with circumferential velocity 5.0 m/s. To the reaction mixture, a solution of ER-804030 (367 g, 231 mmol) in tetrahydrofuran (6970ml) was added dropwise over 1hr at 28 °C. After completion of addition, the mixture was stirred for 1 hour with circumferential velocity 2.67 m/s at the same temperature. To the reaction mixture, heptane (7340ml) was added. To the reaction mixture, 10% citric acid aqueous solution (3670g) was added and stirred for 15 minutes at room temperature. The aqueous layer was removed and the layer was re-extracted with heptane (3670ml). The organic layers were combined and washed with aqueous solution of 5% sodium bicarbonate and 10% sodium sulfate (3000ml). The organic layer was concentrated to afford ER-413207 (322 g, 220 mmol, yield 95.2%).

### [Example 3] Manufacturing of ER-118047/48

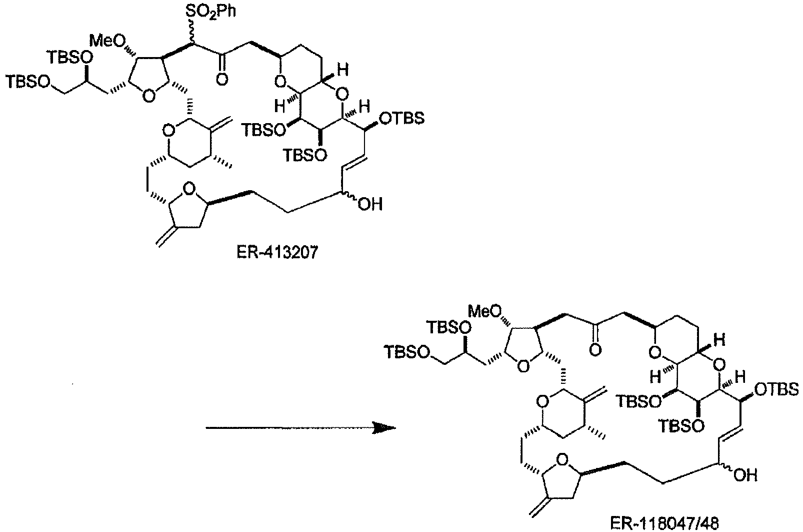

A homogenizer (Body; IKA T25 digital ULTRA TURRAX, Shaft; IKA S25N-25F) was used to stir the reaction.

To a reaction vessel, ER-413207 (5.0 g, 3.45 mmol), chromium chloride (III) 6 hydrate (1.1 g), 4,4'-di-tert-butyl-2,2'-bipyridyl (1.1 g) and manganese (powder, particle size;75 micrometer, 751 mg) were added, and then the reaction vessel was moved in a glove box. Tetrahydrofuran (100 mL) was added to the reaction vessel, and then stirred for 2 hours by the homogenizer with circumferential velocity 5.34 m/s. The reaction vessel was gotten out of the glove box, then, to the reaction mixture, heptane (100mL) was added. To the reaction mixture, methanol (50mL) and 0.5N hydrochloric acid aqueous solution (50 mL) was added and stirred for 40 minutes at room temperature. The aqueous layer was removed and the layer was re-extracted with heptane (100mL). The organic layers were combined and washed with aqueous solution of 5% sodium bicarbonate and 10% sodium sulfate (50mL). The organic layer was concentrated to afford ER-118047/48 (4.5 g, 3.43 mmol, yield 96.6%).

### [Example 4] Manufacturing of ER-118047/48

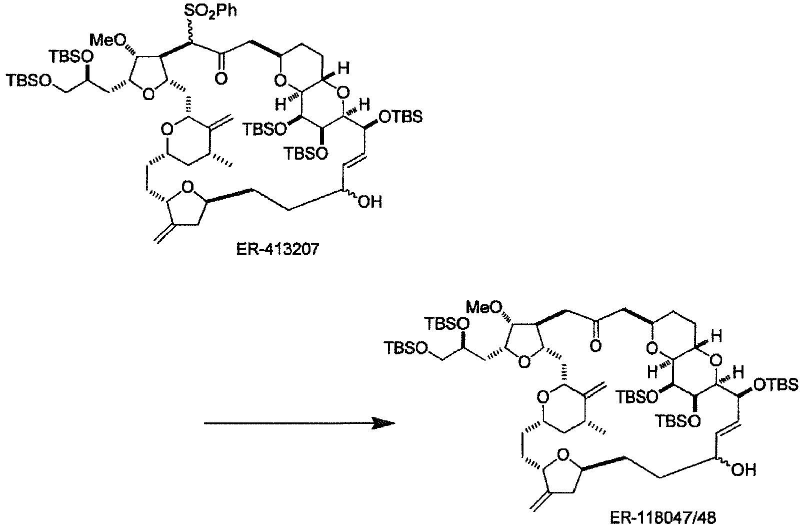

A homogenizer (Body; IKA T25 digital ULTRA TURRAX, Shaft; IKA S25N-25F) was used to stir the reaction.

To a reaction vessel, ER-413207 (12.0 g, 8.21 mmol), chromium chloride (III) 6 hydrate (2.6 g), 4,4'-di-tert-butyl-2,2'-bipyridyl (2.6 g) and manganese (powder, particle size;75 micrometer, 1.8 g) were added, and then the reaction vessel was moved in a glove box. Tetrahydrofuran (240 mL) was added to the reaction vessel, and then stirred for 2 hours and 30 minutes by the homogenizer with circumferential velocity 2.85 to 4.63 m/s. The reaction vessel was gotten out of the glove box, then, to the reaction mixture, heptane (240mL) was added. To the reaction mixture, methanol (60 mL) and 10% citric acid aqueous solution (120 mL) was added and stirred for 45 minutes at room temperature. The aqueous layer was removed and the organic layer was washed with aqueous solution of 5% sodium bicarbonate and 10% sodium sulfate (120 mL). The organic layer was concentrated to afford ER-118047/48 (10.2 g, 7.74 mmol, yield 94.3%).

### [Example 5] Manufacturing of ER-118047/48

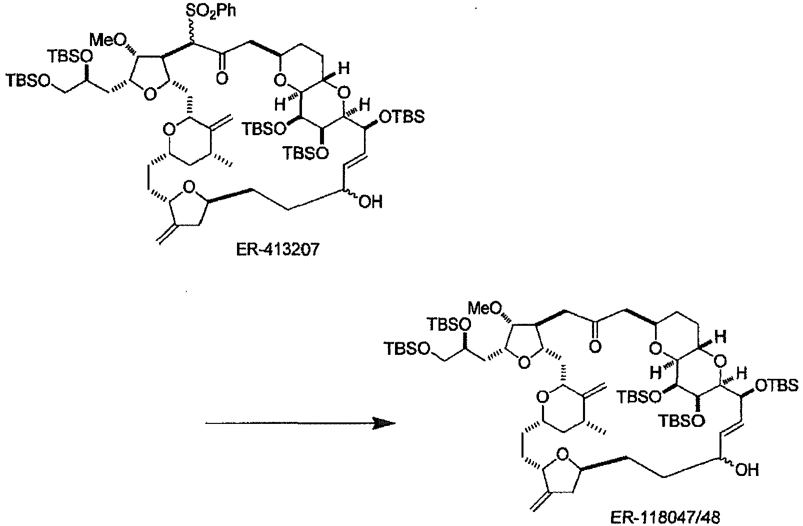

A homogenizer (Body; IKA T65 digital ULTRA TURRAX, Shaft; IKA S65KG-HH-G65F) was used to stir the reaction.

A reaction vessel with chromium chloride (III) 6 hydrate (71.5 g), 4,4'-di-tert butyl-2,2'-bipyridyl (72.0 g) and manganese (powder, particle size;75 micrometer, 49.2 g) was filled with argon gas. To the reaction vessel, tetrahydrofuran (3000 mL) was added. To the reaction mixture, a solution of ER-413207 (327 g, 223 mmol) in tetrahydrofuran (3500mL) was added, and then stirred for 5 hours by the homogenizer with circumferential velocity 5.0 to 7.6 m/s. To the reaction mixture, heptane (6000 mL) was added. To the reaction mixture, methanol (1640 mL) and 10% citric acid aqueous solution (3270 g) was added and stirred for 1 hour at room temperature. The aqueous layer was removed. The organic layer was washed with aqueous solution of 5% sodium bicarbonate and 10% sodium sulfate (3270 g). The organic layer was concentrated to afford ER-118047/48 (279 g, 211 mmol, yield 94.3%).

### [Comparative Example 1] Manufacturing of ER-413207

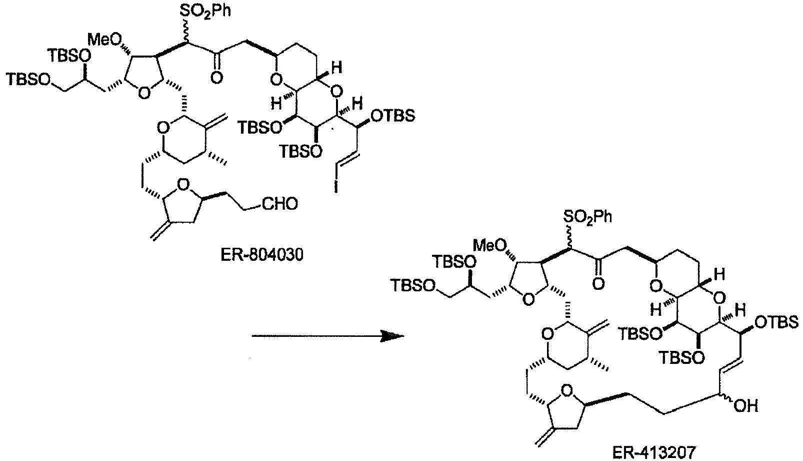

A reaction vessel with chromium chloride (III) (29.9 mg), 4,4'-di-tert-butyl-2,2'-bipyridyl (50.7 mg), zirconocene chloride (607.0 mg) and manganese (powder, particle size;75 micrometer, 415.0 mg) was filled with argon gas. To the reaction vessel, tetrahydrofuran (30 mL) was added, and then stirred for 8 hours by a stirring blade with 450 rpm. To the reaction mixture, a suspension of Ni-neo complex (63.0 mg) in tetrahydrofuran (5 ml) was added and then stirred for 10 minutes by the stirring blade with 450 rpm. To the reaction mixture, a solution of ER-804030 (3.0 g, 1.91 mmol) in tetrahydrofuran (85 ml) was added dropwise over 15 minutes at room temperature. After completion of addition, the mixture was stirred for 11 hours by the stirring blade with 450 rpm at the same temperature. To the reaction mixture, heptane (60 ml) and 10% citric acid aqueous solution (30 mL) were added and stirred for 40 minutes at room temperature. The aqueous layer was removed and the layer was re-extracted with heptane (30ml). The organic layers were combined and washed with aqueous solution of 5% sodium bicarbonate and 10% sodium sulfate (30ml). The organic layer was concentrated to afford ER-413207 (2.6 g, 1.75 mmol, yield 92.8%).

### [Comparative Example 2] Manufacturing of ER-118047/48

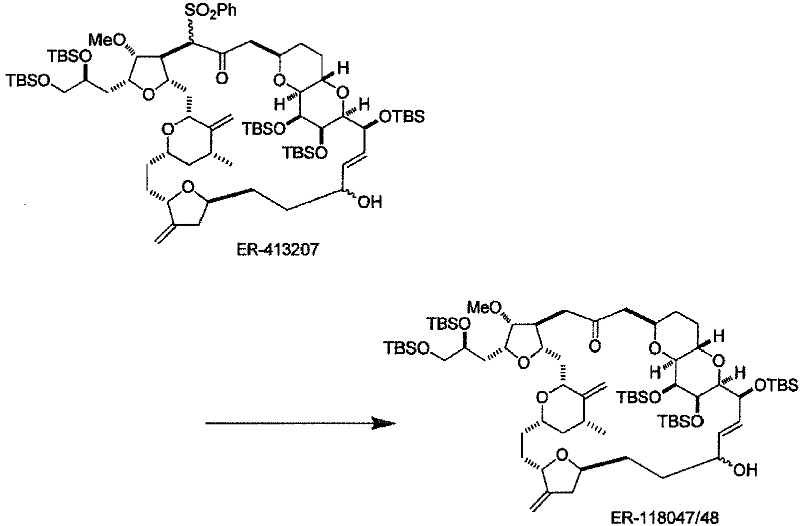

In a glove box, to a reaction vessel, ER-413207 (1.5 g, 1.03 mmol), chromium chloride (III) 6 hydrate (302 mg), 4,4'-di-tert-butyl-2,2'-bipy-ridyl (304 mg), manganese (powder, particle size;75 micrometer, 208 mg) and tetrahydrofuran (30mL) were added, and then the reaction vessel was gotten out of the glove box. After stirring for 9 hours by the stirring blade with 500 rpm, it was stirred for another 3 hours with 550 rpm, and further stirred for another 13 hours with 750 rpm. To the reaction mixture, heptane (30 mL) and 0.5N hydrochloric acid aqueous solution (15 mL) were added and stirred for 1 hour at room temperature. The aqueous layer was removed and the layer was re-extracted with heptane (30 mL). The organic layers were combined and washed with aqueous solution of 5% sodium bicarbonate and 10% sodium sulfate (15 mL). The organic layer was concentrated to afford ER-118047/48 (1.0 g, 0.763 mmol, yield 74.1%).

### [Manufacturing Examples]

Eribulin represented by the formula (I) was manufactured according to the manufacturing examples as follows. And Eribulin mesylate can be obtained from ER-086526 which is obtained by the manufacturing example 4 according to the aforementioned method of step E.

### [Manufacturing Example 1]

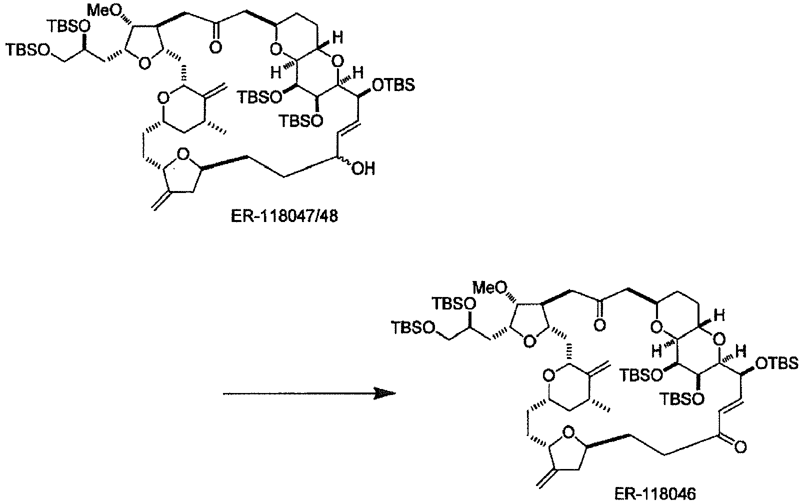

Allyl alcohol ER-118047/48 was dissolved in dichloromethane (0.04 wt % water, 9 vol) and the reactor was placed in a water bath (20 °C) and the solution was treated with Dess-Martin Reagent (0.48 wt, 1.5 eq). The reaction mixture was treated with saturated aqueous sodium bicarbonate (9 vol) and 10 wt % aqueous sodium sulfate (9 vol) then stirred for 20 minutes and transferred to a separatory funnel with DCM (10 vol). The aqueous layer was discarded, and the organic layer was evaporated to obtain a residue. The crude material was purified by flash chromatography (prepped with 3 CV (1:1 (V/V) DCM/heptane, the material was loaded with 1:1 DCM/heptane then eluted with 10/10/1 heptane/DCM/MTBE). The product-containing fractions were concentrated and stored under inert atmosphere at -20 °C.

### [Manufacturing Example 2]

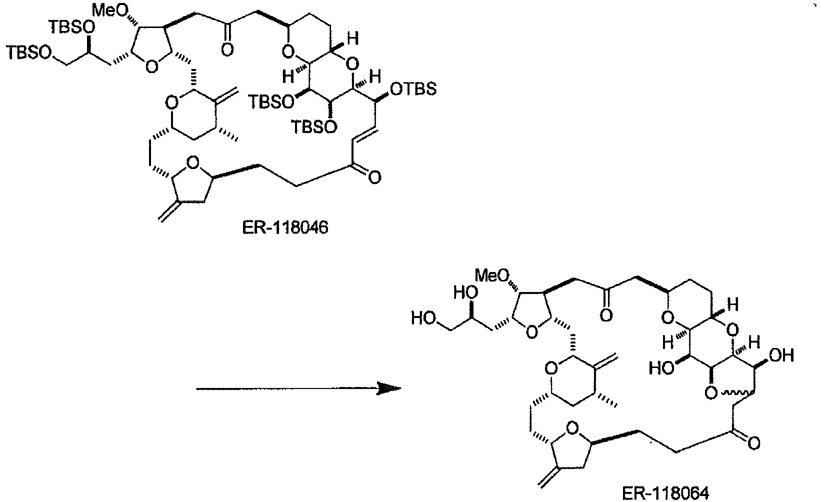

Into an appropriately sized reaction vessel (Vessel A) was charged imidazole hydrochloride (0.39 wt, 5 eq) followed by 1 M TBAF in THF (7.6 vol, 10 eq) at ambient temperature. The resulting mixture was stirred until it was homogeneous (15-30 minutes). Into a second reaction vessel (Vessel B) was charged ER-118046 (1 wt, 1 eq) and THF (33 vol). The contents of Vessel B were placed under an inert atmosphere and stirred until ER-118046 was fully dissolved. The contents of Vessel A (TBAF/Imidazole) were charged as a single portion into Vessel B (ER-118046/THF). After 3-4 days, the reaction solution was loaded onto a column and purified by silica gel chromatography.

### [Manufacturing Example 3]

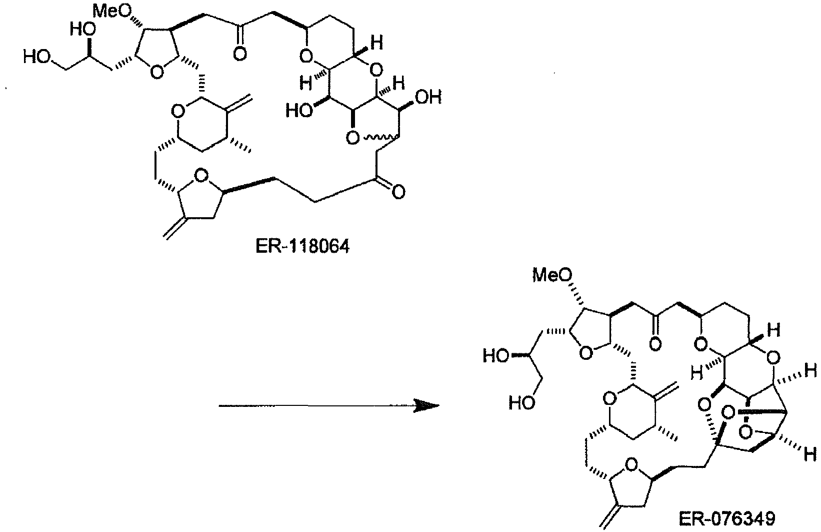

The dried ER-118064 residue was dissolved in anhydrous dichloromethane (28 vol) under a nitrogen atmosphere and treated with PPTS (1.0 wt, 5.2 eq) in one portion. After 30-90 minutes, the reaction mixture was directly loaded atop of a column in an appropriate manner and purified by silica gel chromatography. The desired fractions of ER-076349 were concentrated in vacuo. The material resulting from the concentration was azeotroped twice from toluene (20 vol), affording ER-076349 as a crunchy colorless solid/foam (0.44 wt, 0.79 eq after collection of residual toluene).

### [Manufacturing Example 4]

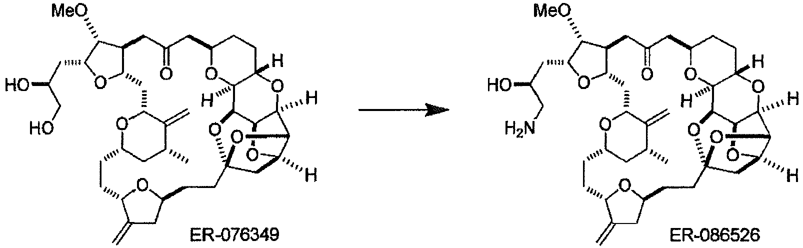

In a clean dry reaction vessel (Vessel C) ER-076349(1 wt, 1 eq) was dissolved in anhydrous toluene (20 vol) and concentrated to dryness under reduced pressure. The substrate was re-dissolved in anhydrous toluene (20 vol) and concentrated to dryness. The substrate was dissolved in DCM (5 vol), and the solution was placed under an argon atmosphere. Collidine (0.66 wts, 4.0 eq) was added as a single portion. Pyridine, as a solution in DCM (Vessel B), was added as a single portion (5 mole %). The resulting mixture in Vessel C was cooled to an internal temperature of - 20 to - 25 °C. A DCM solution of Ts₂O (1.02 eq) was added dropwise while the internal temperature was maintained below - 16 °C. The reaction was stirred at - 20 to - 25 °C for 80 minutes then warmed to 0 °C over 20 minutes and stirred for an additional 20 minutes. The reaction was quenched with water (2 vol). The bath was removed, and the reaction allowed to warm to room temperature (15-20 °C) and stirred (20 minutes). The reaction was rinsed to a larger vessel using the IPA (100 vol) and aqueous ammonium hydroxide (100 vol) was added to the reaction. The reaction was stirred at room temperature for 15-36 hours, monitoring for the disappearance of the tosylate (ER-082892) and epoxide (ER-809681) which formed in situ. The reaction was concentrated to dryness or near dryness at reduced pressure. The resulting material was diluted with DCM (25-40 vol) and washed pH 10 buffer (NaHCO₃/Na₂CO₃ (aq), 10 vol). The aqueous phase was back extracted with 25 vol of DCM and the combined organic layers were concentrated to dryness. The resulting free amine was purified by silica gel chromatography using a buffered ACN/water mobile phase. The pooled fractions were concentrated at reduced pressure to remove ACN. The resulting aqueous layer was diluted with DCM (40 vol) and with 30 vol of a pH 10 buffered stock solution (NaHCO₃/Na₂CO₃). The layers were mixed well and separated. The aqueous phase was back extracted with 25 vol of DCM and the combined organic layers were concentrated to dryness. The resulting free amine was filtered as a solution in 3:1 DCM/pentane and concentrated to dryness (0.80 wts) to afford ER-086526.

Example 1 and 2 showed that the time to complete the reactions is 1 hour and the reactions have completed within shorter time than comparative Example 1. Furthermore, as shown in Example 3 to 5, the reactions have completed within shorter time than Comparative Example 2, and also they provide substantial improvements in the yields as 94.3% to 96.6%.

As above, the reaction progressed in short time and in good yield using the homogenizer in the reaction, even though the reaction is such as manufacturing scale far more than the laboratory scale. Thereby this invention provides manufacturing methods of Eribulin and pharmaceutically acceptable salt thereof efficiently and in good yield.

## Claims

1. A method for manufacturing a compound represented by a formula (II) or salt thereof 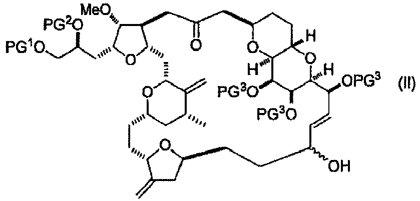
wherein PG¹, PG² and PG³ each independently represent a protective group of a hydroxyl group
from a compound represented by a formula (III), 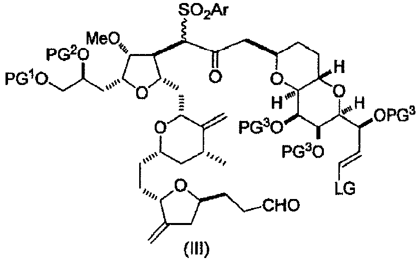
wherein Ar represents an aryl group which may have substituent groups or a heteroaryl group which may have substituent groups, PG¹, PG² and PG³ each independently represent a protective group of a hydroxyl group and LG represents a leaving group,
comprising a reaction step where a reactant is stirred with homogenizer in the presence of manganese powder and chromium compound.

2. The method according to claim 1, wherein the chromium compound is chromium chloride (III).

3. The method according to claim 1 or 2, wherein the step where the reactant is stirred with homogenizer in the presence of manganese powder and chromium compound is a step for manufacturing a compound represented by a formula (IV), 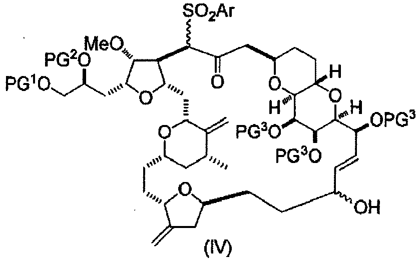
wherein Ar represents an aryl group which may have substituent groups or a heteroaryl group which may have substituent groups, PG¹, PG² and PG³ each independently represent a protective group of hydroxyl group
from a compound represented by a formula (III) 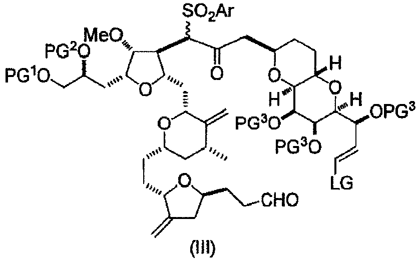
wherein Ar represents an aryl group which may have substituent groups or a heteroaryl group which may have substituent groups, PG¹, PG² and PG³ each independently represent a protective group of a hydroxyl group and LG represents a leaving group.

4. The method according to any one of claim 1 to 3, wherein the step where the reactant is stirred with homogenizer in the presence of manganese powder and chromium compound is a step for manufacturing a compound represented by a formula (II), 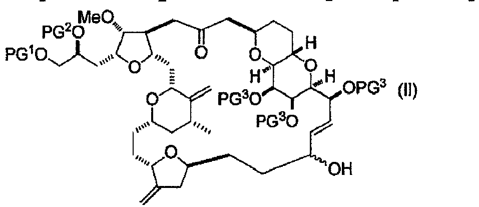
wherein PG¹, PG² and PG³ each independently represent a protective group of hydroxyl group,
from a compound represented by a formula (IV), 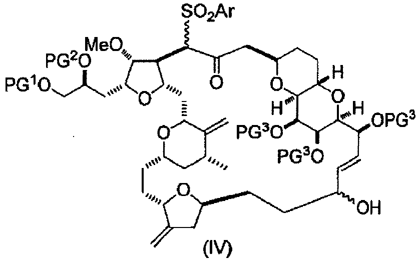
wherein Ar represents an aryl group which may have substituent groups or a heteroaryl group which may have substituent groups, PG¹, PG² and PG³ each independently represent a protective group of hydroxyl group.

5. The method according to claims 1 or 2, wherein the step where the reactant is stirred with homogenizer in the presence of manganese powder and chromium compound is a step for manufacturing a compound represented by a formula (II), 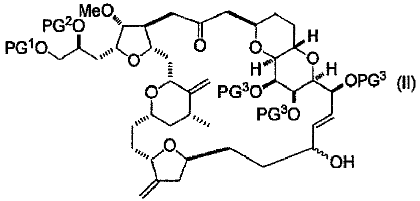
wherein PG¹, PG² and PG³ each independently represent a protective group of hydroxyl group,
from a compound represented by a formula (V), 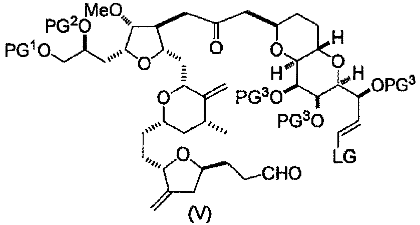
wherein PG¹, PG² and PG³ each independently represent a protective group of a hydroxyl group and LG represents a leaving group.

6. The method according to claims 3 or 5, wherein conducting the reaction in the presence of nickel compound in the reaction step.

7. The method according to any one of claims 1 to 6, wherein catalytic amount of chromium compound is used.

8. The method according to any one of claims 1 to 7, wherein PG¹, PG² and PG³ are tert-butyldimethylsilyl (TBS) group.

9. A method for manufacturing Eribulin or the salt thereof, comprising the method according to any one of claims 1 to 8.

10. The method according to claim 9, wherein the salt of Eribulin is Eribulin mesylate.

## Patentansprüche

1. Verfahren zur Herstellung einer Verbindung der Formel (II) oder eines Salzes davon 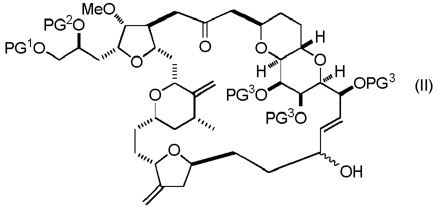
worin PG¹, PG² und PG³ jeweils unabhängig eine Schutzgruppe für eine Hydroxylgruppe darstellen,
aus einer Verbindung der Formel (III), 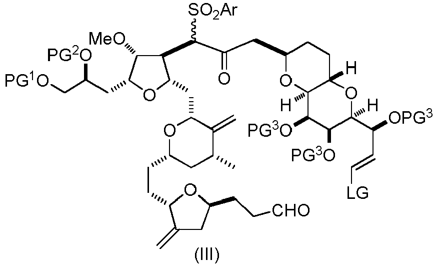
worin Ar eine Arylgruppe, die Substituentengruppen aufweisen kann, oder eine Heteroarylgruppe, die Substituentengruppen aufweisen kann, darstellt, PG¹, PG² und PG³ jeweils unabhängig eine Schutzgruppe für eine Hydroxylgruppe darstellen und LG eine Abgangsgruppe darstellt,
umfassend einen Reaktionsschritt, in dem ein Reaktant mit einem Homogenisator in Gegenwart eines Manganpulvers und einer Chromverbindung gerührt wird.

2. Verfahren gemäss Anspruch 1, wobei die Chromverbindung Chromchlorid (III) ist.

3. Verfahren gemäss Anspruch 1 oder 2, wobei der Schritt, in dem der Reaktant mit einem Homogenisator in Gegenwart eines Manganpulvers und einer Chromverbindung gerührt wird, ein Schritt zur Herstellung einer Verbindung der Formel (IV): 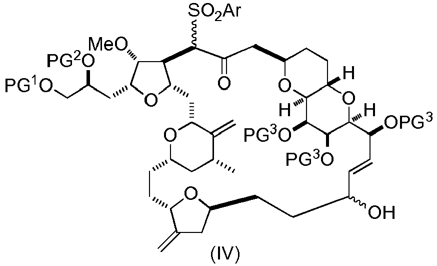
worin Ar eine Arylgruppe, die Substituentengruppen aufweisen kann, oder eine Heteroarylgruppe, die Substituentengruppen aufweisen kann, darstellt, PG¹, PG² und PG³ jeweils unabhängig eine Schutzgruppe für eine Hydroxylgruppe darstellen
aus einer Verbindung der Formel (III) ist: 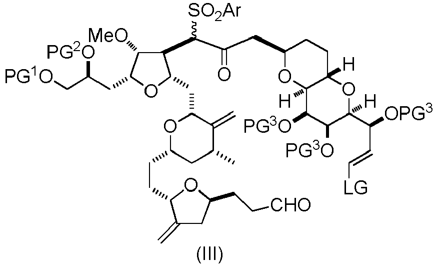
worin Ar eine Arylgruppe, die Substituentengruppen aufweisen kann, oder eine Heteroarylgruppe, die Substituentengruppen aufweisen kann, darstellt, PG¹, PG² und PG³ jeweils unabhängig eine Schutzgruppe für eine Hydroxylgruppe darstellen und LG eine Abgangsgruppe darstellt.

4. Verfahren gemäss irgendeinem der Ansprüche 1 bis 3, wobei der Schritt, in dem der Reaktant mit einem Homogenisator in Gegenwart eines Manganpulvers und einer Chromverbindung gerührt wird, ein Schritt zur Herstellung einer Verbindung der Formel (II): 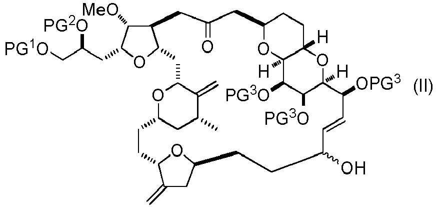
worin PG¹, PG² und PG³ jeweils unabhängig eine Schutzgruppe für eine Hydroxylgruppe darstellen,
aus einer Verbindung der Formel (IV) ist: 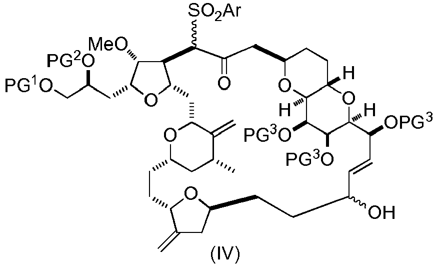
worin Ar eine Arylgruppe, die Substituentengruppen aufweisen kann, oder eine Heteroarylgruppe, die Substituentengruppen aufweisen kann, darstellt, PG¹, PG² und PG³ jeweils unabhängig eine Schutzgruppe für eine Hydroxylgruppe darstellen.

5. Verfahren gemäss Anspruch 1 oder 2, wobei der Schritt, in dem der Reaktant mit einem Homogenisator in Gegenwart eines Manganpulvers und einer Chromverbindung gerührt wird, ein Schritt zur Herstellung einer Verbindung der Formel (II): 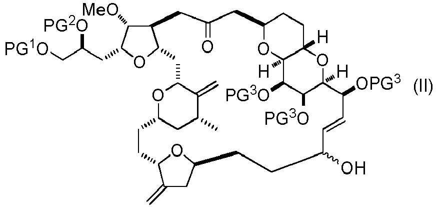
worin PG¹, PG² und PG³ jeweils unabhängig eine Schutzgruppe für eine Hydroxylgruppe darstellen,
aus einer Verbindung der Formel (V) ist: 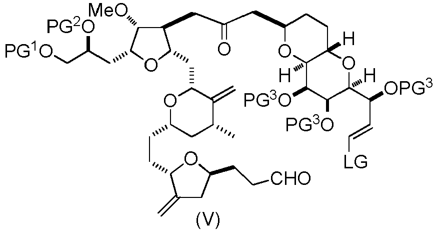
worin PG¹, PG² und PG³ jeweils unabhängig eine Schutzgruppe für eine Hydroxylgruppe darstellen und LG eine Abgangsgruppe darstellt.

6. Verfahren gemäss Anspruch 3 oder 5, wobei die Reaktion in Gegenwart einer Nickelverbindung im Reaktionsschritt durchgeführt wird.

7. Verfahren gemäss irgendeinem der Ansprüche 1 bis 6, wobei eine katalytische Menge der Chromverbindung verwendet wird.

8. Verfahren gemäss irgendeinem der Ansprüche 1 bis 7, wobei PG¹, PG² und PG³ eine tert-Butyldimethylsilyl (TBS)-Gruppe sind.

9. Verfahren zur Herstellung von Eribulin oder dem Salz davon, umfassend das Verfahren gemäss irgendeinem der Ansprüche 1 bis 8.

10. Verfahren gemäss Anspruch 9, wobei das Salz von Eribulin Eribulinmesylat ist.

## Revendications

1. Procédé de fabrication d'un composé représenté par une formule (II) ou sel de celui-ci : 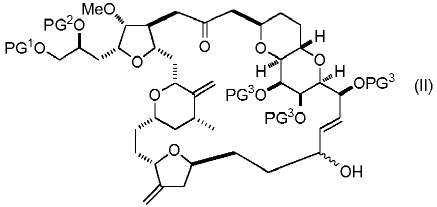
dans laquelle PG¹, PG² et PG³ représentent chacun indépendamment un groupe protecteur d'un groupe hydroxyle
d'un composé représenté par la formule (III), 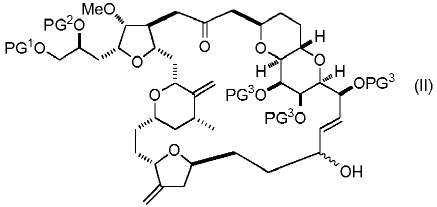
dans laquelle Ar représente un groupe aryle qui peut avoir des groupes substituants ou un groupe hétéroaryle qui peut avoir des groupes substituants, PG¹, PG² et PG³ représentent chacun indépendamment un groupe protecteur d'un groupe hydroxyle et LG représente un groupe partant,
comprenant une étape de réaction dans laquelle un réactif est mélangé avec un homogénéisateur en présence de poudre de manganèse et de composé chrome.

2. Le procédé selon la revendication 1, dans lequel le composé chrome est le chlorure de chrome (III).

3. Le procédé selon la revendication 1 ou 2, dans lequel l'étape dans laquelle le réactif est mélangé avec un homogénéisateur en présence de poudre de manganèse et de composé chrome est une étape de fabrication d'un composé représenté par une formule (IV), 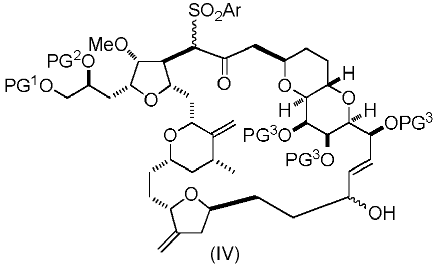
dans laquelle Ar représente un groupe aryle qui peut avoir des groupes substituants ou un groupe hétéroaryle qui peut avoir des groupes substituants, PG¹, PG² et PG³ représentent chacun indépendamment un groupe protecteur d'un groupe hydroxyle
d'un composé représenté par la formule (III) 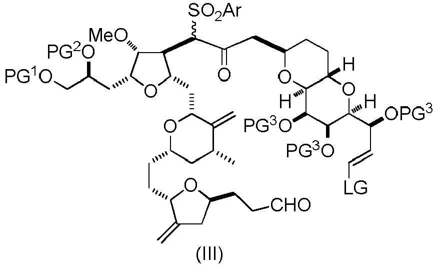
dans laquelle Ar représente un groupe aryle qui peut avoir des groupes substituants ou un groupe hétéroaryle qui peut avoir des groupes substituants, PG¹, PG² et PG³ représentent chacun indépendamment un groupe protecteur d'un groupe hydroxyle et LG représente un groupe partant.

4. Le procédé selon l'une quelconque des revendications 1 à 3, dans lequel l'étape dans laquelle le réactif est mélangé avec un homogénéisateur en présence de poudre de manganèse et de composé chrome est une étape de fabrication d'un composé représenté par une formule (II), 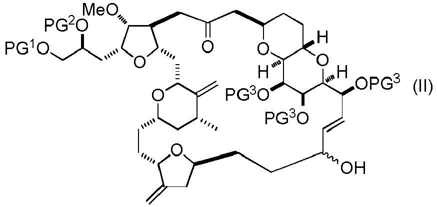
dans laquelle PG¹, PG² et PG³ représentent chacun indépendamment un groupe protecteur d'un groupe hydroxyle
d'un composé représenté par la formule (IV), 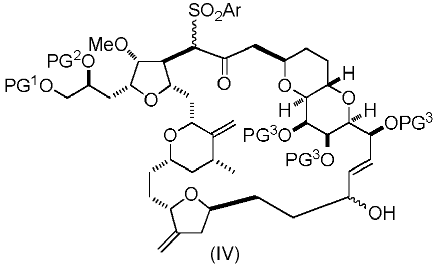
dans laquelle Ar représente un groupe aryle qui peut avoir des groupes substituants ou un groupe hétéroaryle qui peut avoir des groupes substituants, PG¹, PG² et PG³ représentent chacun indépendamment un groupe protecteur d'un groupe hydroxyle.

5. Le procédé selon l'une quelconque des revendications 1 à 2, dans lequel l'étape dans laquelle le réactif est mélangé avec un homogénéisateur en présence de poudre de manganèse et de composé chrome est une étape de fabrication d'un composé représenté par une formule (II), 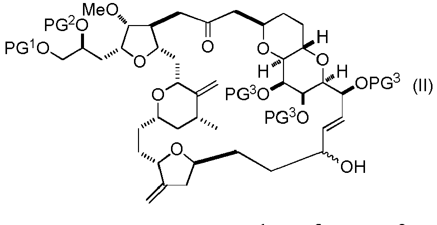
dans laquelle PG¹, PG² et PG³ représentent chacun indépendamment un groupe protecteur d'un groupe hydroxyle
d'un composé représenté par la formule (V), 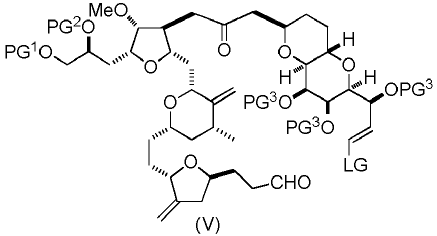
dans laquelle PG¹, PG² et PG³ représentent chacun indépendamment un groupe protecteur d'un groupe hydroxyle et LG représente un groupe partant.

6. Le procédé selon les revendications 3 ou 5, dans lequel la réaction est réalisée en présence de composé nickel dans l'étape de réaction.

7. Le procédé selon l'une quelconque des revendications 1 à 6, dans lequel une quantité catalytique de composé chrome est utilisée.

8. Le procédé selon l'une quelconque des revendications 1 à 7, dans lequel PG¹, PG² et PG³ sont un groupe tert-butyldiméthylsilyle (TBS).

9. Procédé de fabrication d'Éribuline ou le sel de celle-ci, comprenant le procédé selon l'une quelconque des revendications 1 à 8.

10. Le procédé selon la revendication 9, dans lequel le sel d'Éribuline est le mésylate d'Éribuline.
